# EUROPEAN PATENT APPLICATION

(11) **EP 2 535 049 A1**
(43) Date of publication of application: **19.12.2012**
(21) Application number: 11382205.0
(22) Date of filing: 17.06.2011
(51) Int. Cl.: A61K 31/519, A61P 25/00, A61P 25/28

(54) **Tadalafil for the treatment of dementia**

(71) Applicant: Proyecto de Biomedicina Cima, S.L., 31008 Pamplona - Navarra (ES); Universidad de Navarra, 31009 Pamplona (Navarra) (ES)
(72) Inventor: Aguirre García, Norberto, 31008 PAMPLONA (ES); Cuadrado Tejedor, María del Mar, 31008 PAMPLONA (ES); Franco Fernández, Rafael, 31008 PAMPLONA (ES); García Osta, Ana María, 31008 PAMPLONA (ES)
(74) Representative: Ponti Sales, Adelaida

(57) **Abstract**

The present invention relates to tadalafil, pharmaceutically aceptable salts and solvates thereof, for use in the prevention or treatment of dementia and Alzheimer's disease in a geriatric patient. The present invention also relates to the use of tadalafil, pharmaceutically aceptable salts and solvates thereof, for the manufacture of a medicament for the prevention or treatment of dementia and Alzheimer's disease in a geriatric patient. These uses of tadalafil comprise the administration to a geriatric patient of an amount effective to prevent or combat dementia and Alzheimer's disease. The invention also relates to a method of preventing or treating dementia and Alzheimer's disease in a geriatric patient, comprising administering to said patient an effective amount of tadalafil.

## Description

### Field of the invention

The present invention is in the field of pharmacology.

### Background of the invention

Dementia is one of the main health and socioeconomical problems of industrialized countries with high life expectancy. Dementia is an acquired organic mental disorder with loss of intellectual abilities of sufficient severity to interfere with social or occupational functioning. The dysfunction is multifaceted and involves memory, behavior, personality, judgment, attention, spatial relations, language, abstract thought, and other executive functions. The intellectual decline is usually progressive, and initially spares the level of consciousness.

Alzheimer's disease (AD) is the most common form of dementia among older people and refers to dementia that does not have an antecedent cause, such as stroke, brain trauma, or alcohol. The research on the treatment of AD is mostly focused on reducing amyloid plaques but, unfortunately, the variety of clinical trials undertaken to date have failed. Therefore, focus is placed now on other hallmarks of the disease such as hyperphosphorylation of the cytoskeletal protein tau.

Tadalafil is a second-generation phosphodiesterase (PDE5) inhibitor currently marketed in pill form for treating erectile dysfunction (Cialis®) and for the treatment of pulmonary arterial hypertension (Adcirca®). PDE5 inhibitors including sildenafil, vardenafil, and tadalafil are rapidly absorbed in the gastrointestinal tract, and their half-life in blood serum is 3.4 hours for vardenafil and sildenafil, and 18 hours for tadalafil (Kulkarni and Patil 2004; Forgue *et al.*, 2006). Tadalafil is consequently reported to act rapidly and its effects are longer than those of sildenafil (Forgue *et al.*, 2006).

Interestingly, and in contrast to what has been described for sildenafil and vardenafil, it is considered that tadalafil cannot display central nervous system actions. According to the scientific discussion for the approval of Cialis® by European Medicines Agency (EMEA), available as one of the marketing authorization documents (WC500026314, page 5 - published on 15/10/2005), it is explicitly reported that "Tadalafil was not observed in the central nervous system". In addition, tadalafil has recently been commercialized as Adcirca® to treat adults with pulmonary arterial hypertension; and, its corresponding EMEA assessment report (WC500069602 - published on 22/01/2010) explicitly describes that "there were no effects on central nervous system (CNS) function at doses ≤100 mg/kg orally." Therefore, tadalafil is reported to both not crossing the blood-brain barrier (BBB) (Cialis® assessment) and not producing functional effects on the CNS (Adcirca® assessment).

Additionally, the recent patent application W02010/074783, which relates to methods for treating conditions associated with amyloid-beta peptide deposit accumulations by administering a phosphodiesterase-binding compound to a subject, reports on page 109 that "[...] the study of fear memory was repeated using tadalafil which is unable to cross the BBB (clogP=1.43 and information from its manufacturer);" which information supports the lack of CNS activity observed and claimed for tadalafil in the patent, where sildenafil is active. Noteworthy, the mice tested were all young.

Similarly, WO2009/124119 relates to methods for treating conditions associated with amyloid-beta peptide deposit accumulations by administering a phosphodiesterase-binding compound to a subject. In one embodiment, the compound is a PDE5 inhibitor [para. 121] selected from sildenafil, tadalafil, vardenafil [para 226]. However, the inventors report that the lack of *in vivo* clinical effects of tadalafil is due to its unability to cross the BBB. Noteworthy too, the mice tested were all young.

Surprisingly and against all established prejudice in the art, the inventors have advantageously found that tadalafil is able to cross the BBB and more importantly, is able to effect pharmacological actions in the tested CNS diseases, particularly dementia and AD, in a geriatric population.

WO2006/110588 relates to compounds, compositions, and methods useful for (i) treating or preventing mild cognitive impairment, or (ii) delaying the progression from mild cognitive impairment to Alzheimer's disease. The compounds are, among other, phosphodiesterase V inhibitors selected from sildenafil, vardenafil, and tadalafil. The only experimental proof provided in this patent disclosure is the effect of rolipram on contextual learning in a murine model of mild cognitive impairment. This disclosure explicitly makes a distinction between mild cognitive impairment and AD by pointing to the different pathophysiology of these two distinct clinical indications.

Baek *et al.* (2011) disclose that tadalafil improves depressive symptoms, ameliorates memory impairment, as well as suppresses apoptosis and enhances cell proliferation in the hippocampus of maternal-separated rat pups. As such Baek *et al.* suggest that tadalafil may be used as a therapeutic strategy for stress-induced neuropsychiatric disorders during the developmental stage. However, the study is restricted to a young population of rats, far from the human senile typical populations of AD. Additionally, it is known that rat pups do not have a mature or robust BBB, and the study is not concerned with taladafil overcoming the BBB.

Zhang *et al.* (2006) disclose that tadalafil improves neurological functional recovery in a rat model of embolic stroke. However, the teachings of Zhang *et al.*, which depart from an embolic stroke physiopathology, are not concerned with the clinical field of AD since it is general common knowledge that AD is a dementia that does not have an antecedent cause, such as stroke, brain trauma or alcohol (Pharmacology. 4th Edition Rang, Dale, Ritter 1999, page 504).

Ko *et al.* (2009) report that tadalafil improves short-term memory by suppressing ischemia-induced apoptosis of hippocampal neuronal cells in gerbils. These teachings depart from an ischemia-induced physiopathological status, and are therefore not concerned with the field of dementia and AD since these conditions do not have an antecedent cause, such as stroke, brain trauma or alcohol as indicated above.

WO2009/133128 relates to combination compositions and methods for the treatment of Alzheimer's disease and related disorders. Although combinations of tadalafil with cilostazol or chlorzoxazone are disclosed, no experimental proof is provided testing or measuring the effect of tadalafil in combination with a second drug in the clinical treatment of AD. The inventors of the present invention have empirically proven that tadalafil, alone, is useful for the treatment of AD. A merely written and speculated effect for a given combination does not allow to logically infer, without incurring into a fallacy, that one of its parts will have this same effect.

### Description of the figures

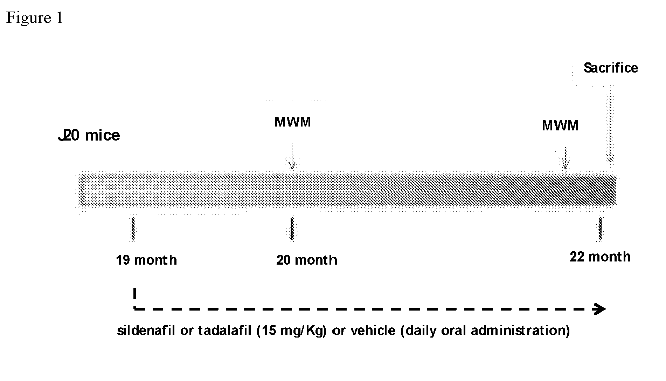

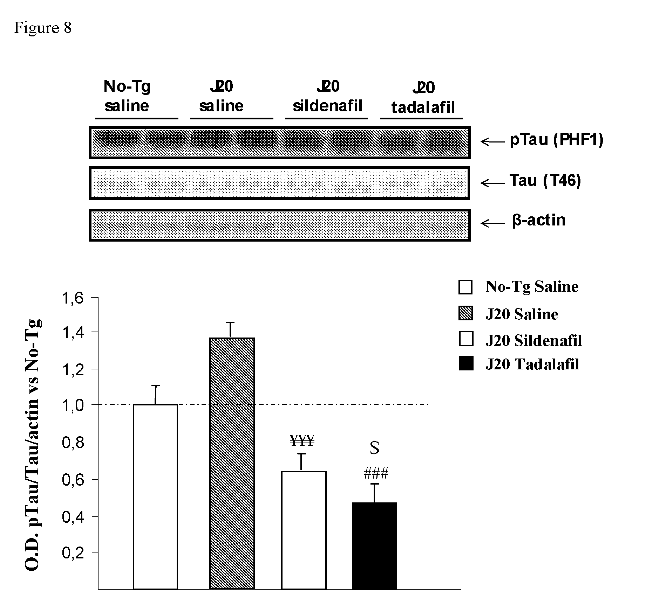

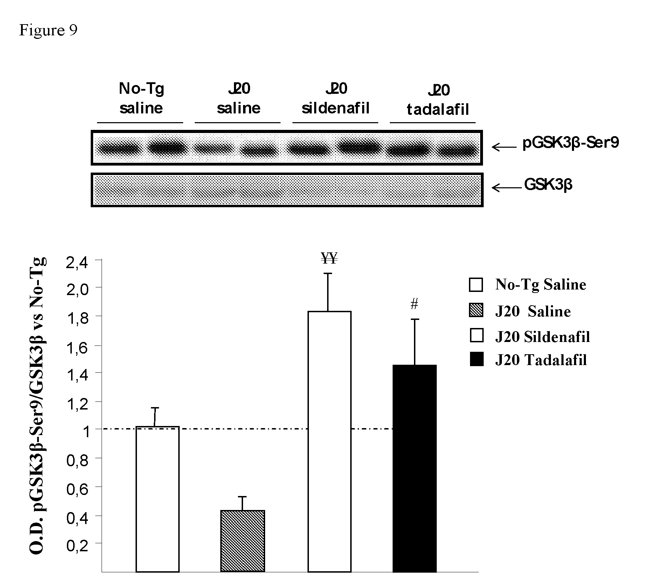

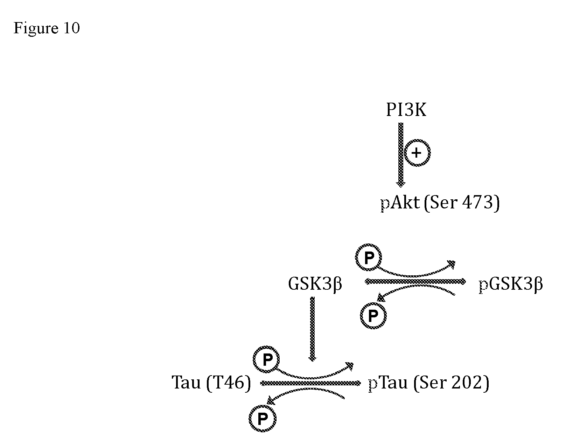

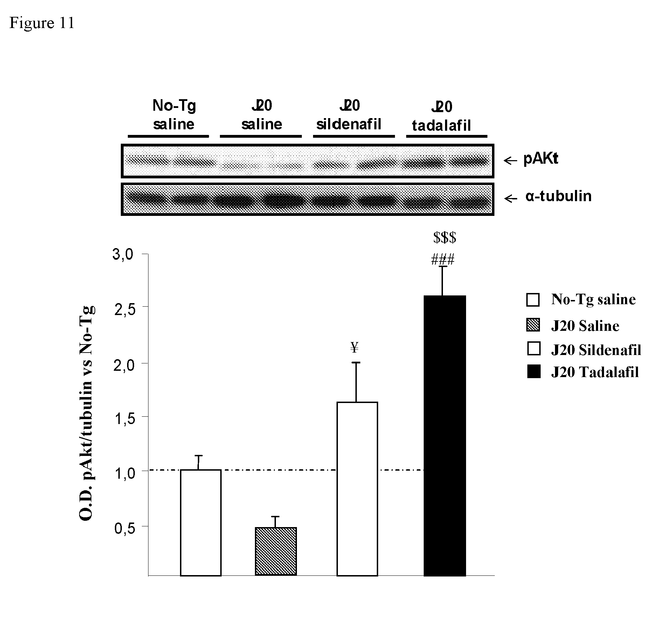

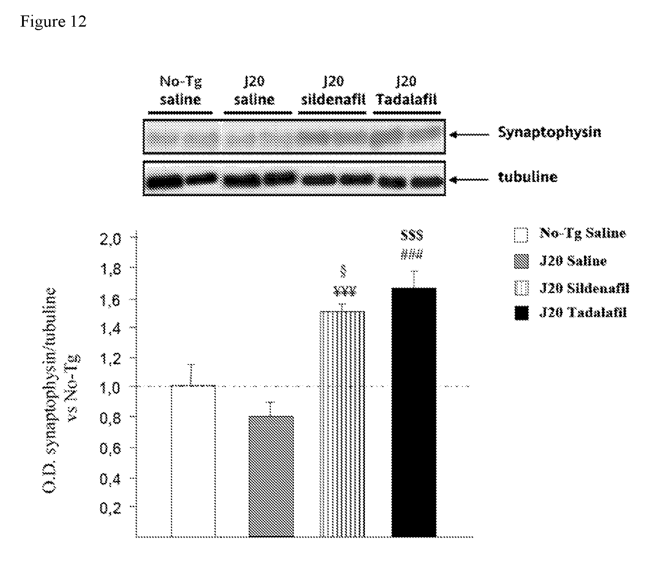

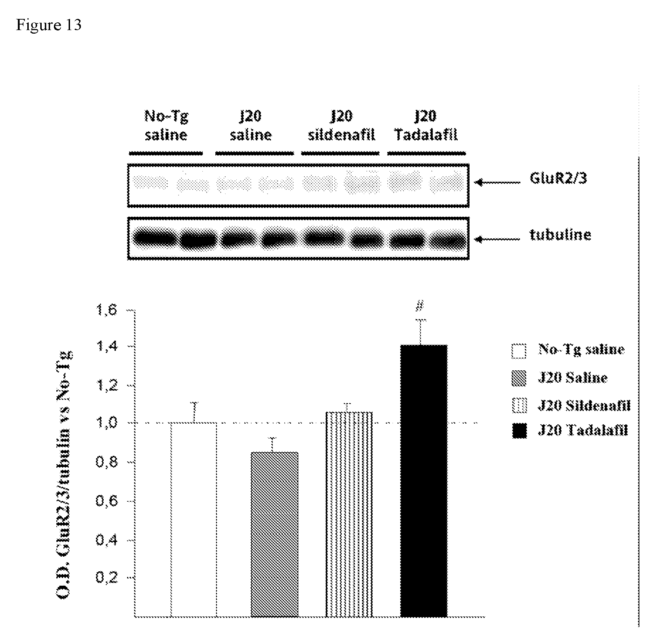

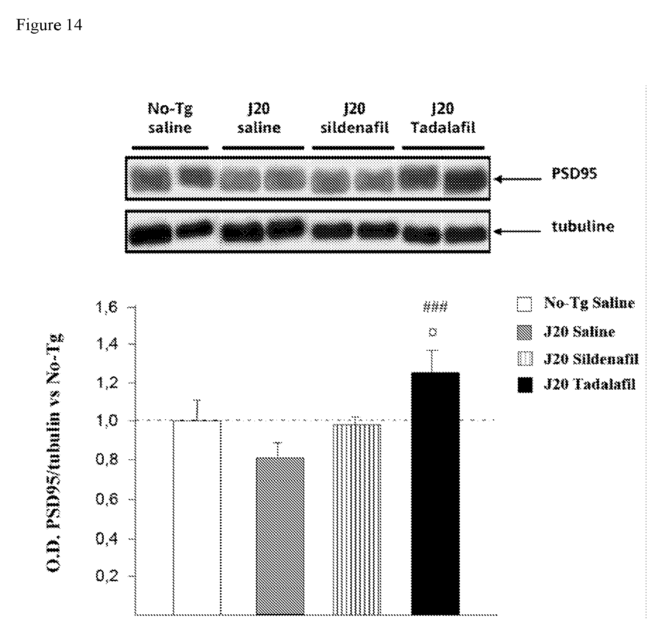

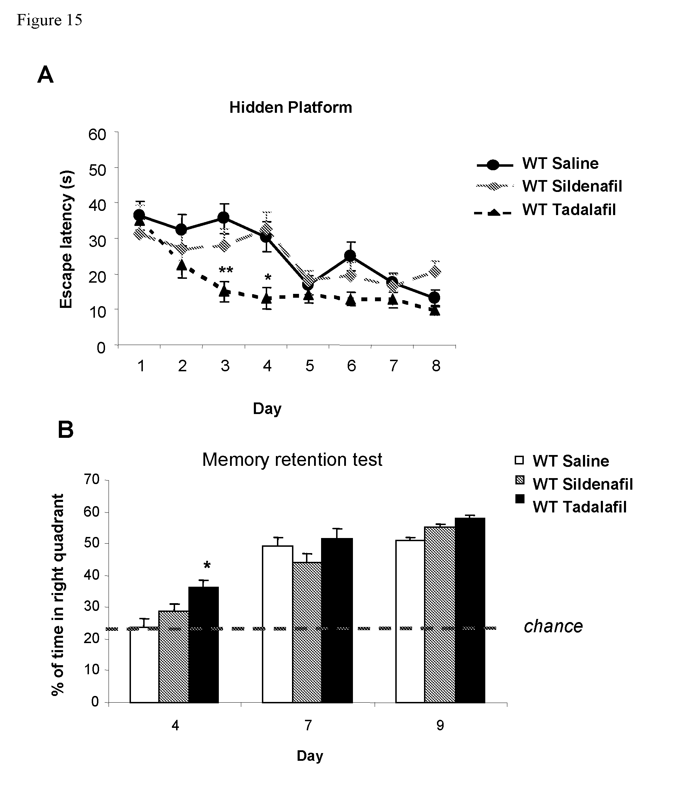

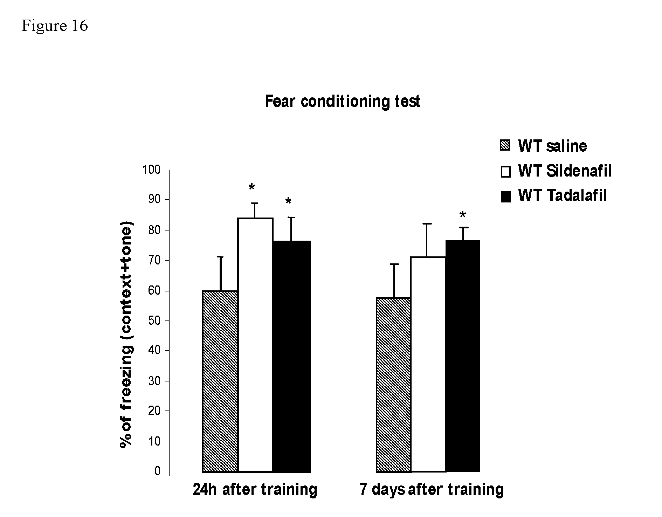

Figure 1. Scheme of the administration of sildenafil, tadalafil, and vehicle.

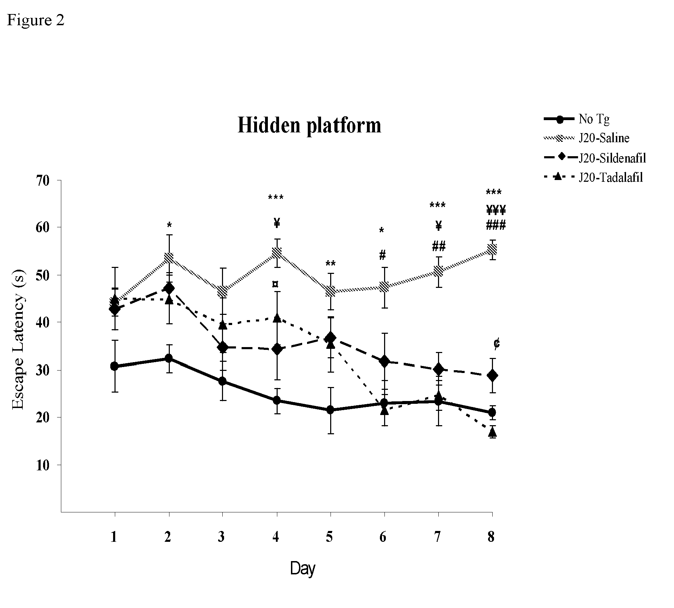

Figure 2. Escape latencies in the invisible platform phase of the test in 21-22 month-old non-transgenic mice (No-Tg) and J20 mice treated for 10 weeks with sildenafil or tadalafil (15mg/Kg) or saline. Values are the mean ± SEM in each group (n=6-8). *p≤0.05, **p≤0.01, ***p≤0.001 No-Tg vs J20-saline; #p≤0.05, ##p≤0.01, ###p≤0.001 J20-Tadalafil vs J20-Saline; ¥p≤0.05, ¥¥¥p≤0.001 J20-Sildenafil vs J20-Saline; 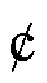 J20-Sildenafil vs J20-Tadalafil. ANOVA followed by Scheffé's test.

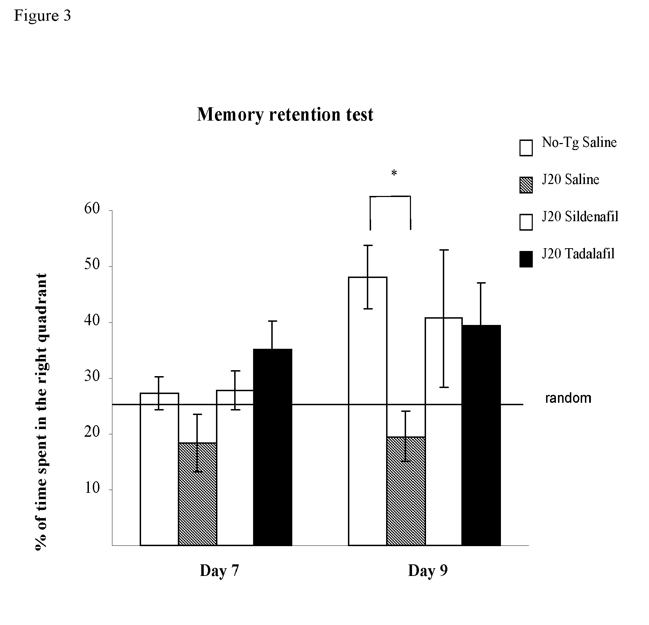

Figure 3. Memory retention test. Percent of time in the right quadrant on days 7 and 9 shown by 21-22 month-old non-transgenic mice (No-Tg) and J20 mice treated for 10 weeks with sildenafil or tadalafil (15mg/Kg) or saline. Values represent the mean ± SEM in each group (n=6-8) (*p≤0.05. ANOVA one factor followed by Scheffé's test).

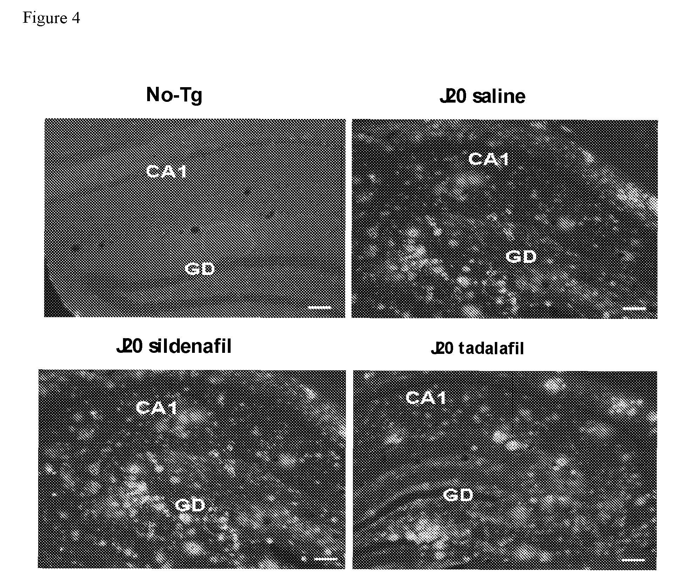

Figure 4. Amyloid plaques in slices of hippocampus from non-transgenic (No-Tg) and J20 mice treated for 10 weeks with saline, sildenafil or tadalafil. Extracellular deposits stained with 6E10 antiserum, which recognizes the 1-17 amino acids of Aβ peptide. Representative images of those obtained from 4 animals per group (4-5 sections per animal) are shown. Scale bar: 100 µm.

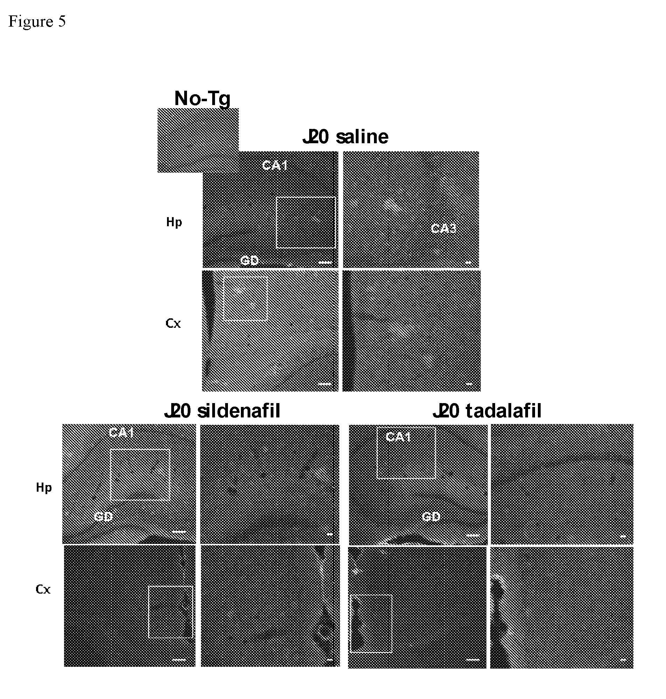

Figure 5. pTau labeling using the PHF-1 antibody in hippocampus or cerebral cortex slices from non-transgenic (No-Tg) and J20 mice treated for 10 weeks with saline, sildenafil or tadalafil. Representative images of those obtained from 4 animals per group (4-5 sections per animal) are shown. Scale bar: 100 µm and 50 µm.

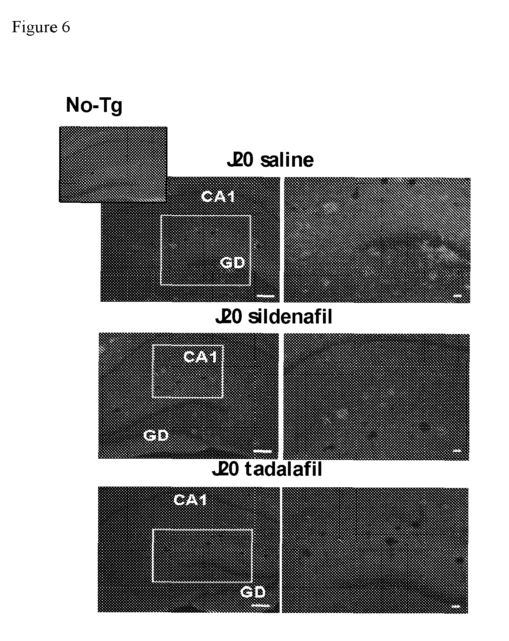

Figure 6. pTau labeling using AT8 antibody in hippocampus slices from non-transgenic (No-Tg) and J20 mice treated for 10 weeks with saline, sildenafil or tadalafil. Representative images of those obtained from 4 animals per group (4-5 sections in per animal) are shown. Scale bar: 100 µm and 50 µm.

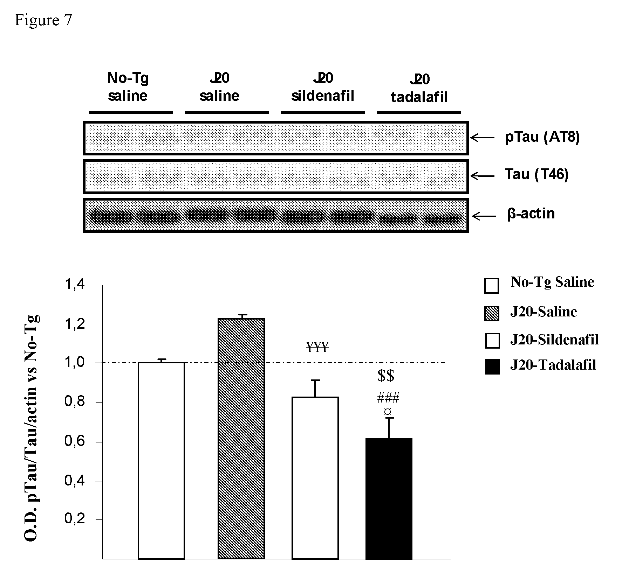

Figure 7. pTau levels in non-transgenic (No-Tg) and J20 animals (22-month-old) treated for 10 weeks with saline, sildenafil or tadalafil. Representative western blot bands made from hippocampal tissues of non-transgenic, and transgenic mice receiving saline, sildenafil or tadalafil are shown. pTau levels (AT8) were normalized to total Tau detected with the T46 antibody referred to actin levels. Values are the mean ± SEM (n=3-4) in relative units of band density. ($$ p≤0,01 tadalafil vs No-Tg; ¥¥¥ p≤ 0.001 sildenafil vs saline; ### p≤0.001 tadalafil vs saline; ¤ p≤0.05 Tadalafil vs Sildenafil. ANOVA followed by Scheffé's test.
Figure 8. pTau levels in non-transgenic and J20 animals (22-month-old) treated for 10 weeks with saline, sildenafil or tadalafil. Representative western blot using hippocampal tissue from non-transgenic (No-Tg), and from transgenic mice receiving saline, sildenafil or tadalafil are shown. pTau levels (PHF1) were normalized to total Tau detected with the T46 antibody referred to actin levels. Values are the mean ± SEM (n=3-4) in relative units of band density. ($ p≤0,05 tadalafil vs No-Tg; ¥¥¥ p≤ 0.001 sildenafil vs saline; ### p≤0.001 tadalafil vs saline. ANOVA followed by Scheffé's test.
Figure 9. pGSK3β-Ser9 levels in non-transgenic (No-Tg) and J20 animals (22-month-old) treated for 10 weeks with saline, sildenafil or tadalafil. Representative western blot using hippocampal tissue from non-transgenic, and transgenic mice receiving saline, sildenafil or tadalafil are shown. pGSK3β-Ser9 levels were normalized to tubulin levels. Values are the mean ± SEM (n=3-4) in relative units of band density. (¥¥ p≤ 0.01 sildenafil vs saline; # p≤0.05 tadalafil vs saline. ANOVA followed by Scheffé's test.
Figure 10. Scheme of Ser-202 pTau signaling pathway.
Figure 11. pAkt levels in non-transgenic (No-Tg) and J20 animals (22-month-old) treated for 10 weeks with saline, sildenafil or tadalafil. Representative western blot using hippocampal tissue from non-transgenic, and transgenic mice receiving saline, sildenafil or tadalafil are shown. pAkt levels were normalized to tubulin levels. Values are the mean ± SEM (n=3-4) in relative units of band density. ($$$ p≤0,05 tadalafil vs No-Tg; ¥ p≤ 0.05 sildenafil vs saline; ### p≤0.05 tadalafil vs saline. ANOVA followed by Scheffé's test.
Figure 12. Synaptophysin levels in non-transgenic (No-Tg) and J20 animals (22-month-old) treated for 10 weeks with saline, sildenafil or tadalafil. Representative western using hippocampal tissue from non-transgenic, and transgenic mice receiving saline, sildenafil or tadalafil are shown. Synaptophysin levels were normalized to tubulin levels. Values are the mean ± SEM (n=3-4) in relative units of band density. Values are the mean ± SEM (n=3-4) in relative units of band density. ($$$ p≤ 0,001 tadalafil vs No-Tg; § p≤ 0.05 sildenafil vs No-Tg; ### p≤0.001 tadalafil vs Saline; ¥¥¥ p≤ 0.001 sildenafil vs saline. ANOVA followed by Scheffé's test.
Figure 13. GluR2/3 levels in non-transgenic (No-Tg) and J20 animals (22-month-old) treated for 10 weeks with saline, sildenafil or tadalafil. Representative western using hippocampal tissue from non-transgenic and transgenic mice receiving saline, sildenafil or tadalafil are shown. GluR2/3 levels were normalized to tubulin levels. Values are the mean ± SEM (n=3-4) in relative units of band density. (# p≤ 0,05 tadalafil vs saline). ANOVA followed by Scheffé's test.
Figure 14. PSD95 levels in non-transgenic (No-Tg) and J20 animals (22-month-old) treated for 10 weeks with saline, sildenafil or tadalafil. Representative western blot using hippocampal tissue from non-transgenic and transgenic mice receiving saline, sildenafil, or tadalafil, are shown. PSD95 levels were normalized to tubulin levels. Values are the mean ± SEM (n=3-4) in relative units of band density. (### p≤ 0,001 tadalafil vs saline; ¤ p≤ 0.05 tadalafil vs sildenafil. ANOVA followed by Scheffé's test.
Figure 15. Morris water maze (MWM) test. (A) Escape latencies in the invisible platform phase in aged wild type (WT) animals treated for 5 weeks with sildenafil, tadalafil (15mg/Kg) or saline. Values are the mean ± SEM in each group (n=6-8). (B) Memory retention test. Percent of time in the right quadrant on days 4, 7 and 9. *p≤0.05, **p≤0.01, tadalafil vs saline. ANOVA followed by Scheffé's test.
Figure 16. Fear conditioning test. Aged wild type (WT) mice receiving sildenafil or tadalafil (15 mg/Kg) for 5 weeks showed a significant enhancement of memory compared to saline treated mice (* p< 0.05 vs saline, ANOVA followed by Scheffé's test). Results are expressed as mean ± SEM; n= 10-12 in each group.

### Summary of the invention

The present invention relates to tadalafil, pharmaceutically aceptable salts and solvates thereof, for use in the prevention or treatment of dementia in a geriatric patient. The present invention also relates to the use of tadalafil, pharmaceutically aceptable salts and solvates thereof, for the manufacture of a medicament for the prevention or treatment of dementia in a geriatric patient. These uses of tadalafil comprise the administration to a geriatric patient of an amount effective to prevent or combat dementia. The invention also relates to a method of preventing or treating dementia in a geriatric patient, comprising administering to said patient an effective amount of tadalafil.

The present invention relates to tadalafil, pharmaceutically aceptable salts and solvates thereof, for use in the prevention or treatment of Alzheimer's disease in a geriatric patient. The present invention also relates to the use of tadalafil, pharmaceutically aceptable salts and solvates thereof, for the manufacture of a medicament for the prevention or treatment of Alzheimer's disease in a geriatric patient. These uses of tadalafil comprise the administration to a geriatric patient of an amount effective to prevent or combat Alzheimer's disease. The invention also relates to a method of preventing or treating Alzheimer's disease in a geriatric patient, comprising administering to said patient an effective amount of tadalafil.

### Disclosure of the invention

The present invention relates to tadalafil, pharmaceutically aceptable salts and solvates thereof, for use in the prevention or treatment of dementia in a geriatric patient. The present invention also relates to the use of tadalafil, pharmaceutically aceptable salts and solvates thereof, for the manufacture of a medicament for the prevention or treatment of dementia in a geriatric patient. These uses of tadalafil comprise the administration to a geriatric patient of an amount effective to prevent or combat dementia. The invention also relates to a method of preventing or treating dementia in a geriatric patient, comprising administering to said patient an effective amount of tadalafil.

The present invention relates to tadalafil, pharmaceutically aceptable salts and solvates thereof, for use in the prevention or treatment of Alzheimer's disease in a geriatric patient. The present invention also relates to the use of tadalafil, pharmaceutically aceptable salts and solvates thereof, for the manufacture of a medicament for the prevention or treatment of Alzheimer's disease in a geriatric patient. These uses of tadalafil comprise the administration to a geriatric patient of an amount effective to prevent or combat Alzheimer's disease. The invention also relates to a method of preventing or treating Alzheimer's disease in a geriatric patient, comprising administering to said patient an effective amount of tadalafil.
Tadalafil refers to the (6*R-trans*)-6-(1,3-benzodioxol-5-yl)-2,3,6,7,12,12a-hexahydro-2-methyl-pyrazino [1',2':1,6] pyrido [3,4-b]indole-1,4-dione, alternatively named (6R, 12aR)-2,3,6,7,12,12a-hexahydro-2-methyl-6-(3,4-methylene-dioxypheny-1)pyrazino[2',1':6.1] pyrido[3,4-b]indole-1,4-dione, as disclosed in Daugan U.S. Pat. No. 5,859,006, and represented by structural formula (I):

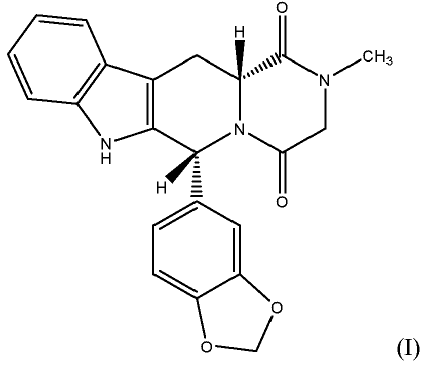

Tadalafil is commercially available from Lilly ICOS, LLC. Methods for its synthesis are described in US Patent No. 5,859,006, the contents of which are hereby incorporated by reference.

For therapeutic use, salts of the compound of formula (I) are those wherein the counter-ion is pharmaceutically acceptable, which salts can be referred to as pharmaceutically acceptable acid and base addition salts. However, salts of acids and bases that are non-pharmaceutically acceptable may also find use, for example, in the preparation or purification of a pharmaceutically acceptable compound. All salts, whether pharmaceutically acceptable or not, are included within the ambit of the present invention.

The pharmaceutically acceptable acid and base addition salts as mentioned hereinabove are meant to comprise the therapeutically active non-toxic acid and base addition salt forms that the compound of formula (**I**) is able to form. The pharmaceutically acceptable acid addition salts can conveniently be obtained by treating the base form with such appropriate acid in an anion form. Appropriate anions comprise, for example, acetate, benzenesulfonate , benzoate, bicarbonate, bisulfate, bitartrate, bromide, calcium edetate, camsyiate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methanesulfonate, methylbromide, methylnitrate, methylsulfate, mucate, napsylate, nitrate, pamoate (embonate), pantothenate, phosphate/diphosphate, polygalacturonate, p-toluenesulfonate, salicylate, stearate, subacetate, succinate, sulfate, tannate, tartrate, teoclate, triethiodide, and the like. Conversely said salt forms can be converted by treatment with an appropriate base into the free base form.

The compound of formula (**I**) containing an acidic proton may also be converted into its nontoxic metal or amine addition salt forms by treatment with appropriate organic and inorganic bases in a cation form. Appropriate basic salts comprise those formed with organic cations such as benzathine, chloroprocaine, choline, diethanolamine, ethylenediamine, meglumine, procaine, and the like; and those formed with metallic cations such as aluminum, calcium, lithium, magnesium, potassium, sodium, zinc, and the like. Conversely said salt forms can be converted by treatment with an appropriate acid into the free form.

The term "solvate" refers to those crystal forms of the compound of the present invention that contain either stoichiometric or non-stoichiometric amounts of solvent. Since water is a solvent, solvates also include hydrates. The term "pseudopolymorph" is synonym to solvate since it applies to polymorphic crystalline forms that have solvent molecules incorporated in their lattice structures. Examples of solvates are hydrates and alcoholates such as methanolates or ethanolates.

Preferably, the free drug is crystalline. However, amorphous and partially amorphous forms of compound (I) also are contemplated, and are included within the present invention.

The term "crystalline" is defined as a form in which the position of the molecules relative to one another is organized according to a three-dimensional lattice structure.

The term "preventing" refers to keep from happening, existing, or alternatively delaying the onset or recurrence of a disease, disorder, or condition to which such term applies, or of one or more symptoms associated with a disease, disorder, or condition. The term "prevention" refers to the act of preventing, as "preventing" is defined immediately above.

The term "treating", as used herein, refers to reversing, alleviating, or inhibiting the progress of the disorder or condition to which such term applies, or one or more symptoms of such disorders or condition. For a given patient, improvement in a symptom, its worsening, regression, or progression may be determined by an objective or subjective measure. Efficacy of treatment may be measured as an improvement in morbidity or mortality (e.g., and without being limited to, lengthening of survival curve for a selected population, improvement in cognition, improvement in quality of life). The term "treatment" refers to the act of treating, as "treating" is defined immediately above.

In one embodiment of the present invention, dementia is presenile or senile.

In one embodiment of the present invention, dementia courses with tauopathy.

In one embodiment of the present invention, dementia is of the Alzheimer's type.

In one embodiment of the present invention, dementia is a moderate dementia of the Alzheimer's type.

In one embodiment of the present invention, dementia is a severe dementia of the Alzheimer's type.

In one embodiment of the present invention, Alzheimer's disease is of moderate grade.

In one embodiment of the present invention, Alzheimer's disease is of moderate to severe grade.

In one embodiment of the present invention, Alzheimer's disease is of severe grade.

In other embodiments, the clinical conditions for which tadalafil, the salts and solvates thereof, is useful, comprise without being limited to Primary Senile Degenerative Dementia, Senile Dementia of Alzheimer Type or Alzheimer Type Senile Dementia, Late Onset Alzheimer Disease, Focal Onset Alzheimer's Disease, Acute Confusional Senile Dementia, Presenile Alzheimer Dementia.

These clinical terms above are conventionally used and known by the skilled in the art, preferably clinicians. Alternatively, their definitions can be found at ICD (International Statistical Classification of Diseases and Related Health Problem), MeSH (Medical Subject Headings of the United States National Library of Medicine).

The term "geriatric" refers to an older population group or patient and is at least 40 or 45 years old, preferably at least 50 years old, more preferably at least 55, 60, or 65 years old.

These ages are to be taken as approximations and not be strictly interpreted.

The term "patient" means animals, in particular mammals such as dogs, cats, cows, horses, sheep, geese, and humans. Particularly preferred patients are mammals, including humans of both sexes.

An "effective amount" of tadalafil and pharmaceutically acceptable salts thereof, may be in the range from 0.1 mg to 500 mg per day, from 1 mg to 500 mg per day, from 2.5 mg to 500 mg per day, from 5 mg to 500 mg per day, from 10 mg to 500 mg per day, from 20 mg to 500 mg per day, from 20 mg to 400 mg per day, from 20 mg to 200 mg per day, from 20 mg to 100 mg per day, preferably from 20 mg to 80 mg per day, more preferably from 20 mg to 40 mg per day.

In one embodiment of the present invention, tadalafil is administered at a dose between 20 and 100 mg once a day. In another embodiment, tadalafil is administered at a dose between 20 and 40 mg once a day.

The term "pharmaceutically acceptable" means that a compound or combination of compounds is sufficiently compatible with the other ingredients of a formulation, and not deleterious to the patient up to those levels acceptable by the industry standards.

Therefore, tadalafil may be formulated into various pharmaceutical forms for administration purposes. As appropriate compositions there may be cited all compositions usually employed for systemically administering drugs. To prepare the pharmaceutical compositions of this invention, an effective amount of tadalafil as the active ingredient is combined in intimate admixture with a pharmaceutically acceptable carrier, which carrier may take a wide variety of forms depending on the manner of preparation desired for administration. These pharmaceutical compositions are desirable in unitary dosage form suitable, particularly, for administration orally, rectally, transdermally, intradermally, topically, by inhalation, or by parenteral routes (i.e., subcutaneous, intravenous, intramuscular or intraperitoneal).

In one embodiment of the present invention, tadalafil is administered orally. Tadalafil can be administered by the oral route in solid dosage forms, such as tablets, capsules, and powders, or in liquid dosage forms, such as elixirs, syrups, and suspensions. The pharmaceutical compositions of this invention can also be administered parenterally, in sterile liquid dosage forms.

In preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed.

Tadalafil may as well be administered in oral dosage forms such as the ones described in patent US7182958, as a free drug in admixture with a diluent, a lubricant, a hydrophilic binder selected from the group consisting of a cellulose derivative, povidone, and a mixture thereof, a disintegrant selected from the group consisting of crospovidone, croscarmellose sodium, and a mixture thereof, and, optionally, microcrystalline cellulose and/or a wetting agent. Optionally, the formulation additionally comprises a second diluent.

Tadalafil may as well be administered as a coprecipitate preparation with a polymer, as disclosed in US5985326, wherein the polymer is for example hydroxypropyl methylcellulose phthalate. This coprecipitate preparation is prepared, then milled, mixed with excipients, and compressed into tablets for oral administration.

Also, free tadalafil drug is preferred in particulate form, and wherein at least 90% of the particles have a particle size of less than about 40 microns, and preferably less than 30 microns. Highly preferred particulate forms of the compound (I) have at least 90% of the particles less than 25 microns in size. Most preferred forms of the free compound (I) are those wherein 90% of the particles are less than 10 microns in size, as described and prepared in US6821975.

Formulations for parenteral administration may be in the form of aqueous or non-aqueous isotonic sterile injection solutions or suspensions. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. These latter suitable additives may be anti-oxidants, preservatives, stabilizing agents, emulsifiers, salts for influencing the osmotic pressure, and/or buffer substances.

In one embodiment of the present invention, tadalafil is administered transdermally. In one embodiment of the present invention, tadalafil is administered topically.

As appropriate topical or transdermal compositions there may be cited for example gels, jellies, creams, pastes, emulsions, dispersions, ointments, films, sponges, foams, aerosols, powders, implants, patches. In the compositions suitable for topical cutaneous administration, the carrier optionally comprises a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a cream or gel.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier Examples of such unit dosage forms are tablets (including scored or coated tablets), capsules, pills, suppositories, powder packets, wafers, injectable solutions or suspensions and the like, and segregated multiples thereof.

Notwithstanding the effective amounts and doses indicated above, still the dose of tadalafil, its pharmaceutically acceptable salts and solvates thereof to be administered will depend on the individual case and, as customary, is to be adapted to the conditions of the individual case for an optimum effect. Thus it depends, of course, on the frequency of administration and on the potency and duration of action of the compound employed in each case for therapy or prophylaxis, but also on the nature and severity of the disease and symptoms, and on the sex, age, weight co-medication and individual responsiveness of the subject to be treated and on whether the therapy is acute or prophylactic. Doses may be adapted in function of weight and for paediatric applications. Daily doses may be administered q.d. or in multiple quantities such as b.i.d., t.i.d. or q.i.d. Alternatively, doses may be administered every other day, every three, every four, every five, every six, every seven days, every other week, every month.

The present invention is illustrated by the following examples, which are not to be construed as limiting.

### Examples

### Tadalafil is able to cross the blood brain barrier

Serum and brain (cortex, striatal and hippocampal) levels of tadalafil after oral administration were determined by high-performance-liquid chromatography separation and mass-spectrometry identification and quantification. The results in Table 1 and 2 clearly indicate tadalafil is able to cross the blood brain barrier. The concentrations attained are high enough to inhibit PDE5 (Ki are in the nanomolar range).

**Table 1. Concentration of tadalafil 90 minutes after oral administration (Cialis), in serum as well as in three different brain regions.**

| Mouse | Serum (µM) | Ctx (µM) | Str (µM) | Hip (µM) |
|---|---|---|---|---|
| 1^{a} | N.D. | N.D. | N.D. | N.D. |
| 2^{b} | 4,95 | 0.61 | 0.39 | 0.45 |
| 3^{b} | 6.53 | 1.25 | 0.82 | 0.91 |
| 4^{b} | 9.65 | 1.22 | 1.09 | 1.54 |
| 5^{b} | 7.27 | 1.35 | 1.09 | 2.19 |
| 6^{b} | 10.23 | 0.73 | 0.72 | 1.48 |
| 7^{b} | 8.10 | 0.91 | 0.93 | 1.35 |

| | | | | |
|---|---|---|---|---|
| The limit of detection of tadalafil in the HPCL-MS conditions was 1 ng ml⁻¹ (0,002 µM) in serum and 10 ng g⁻¹ (0,026 µM) in brain tissue. a) Not treated Mouse; b) mice treated with Cialis, oral administration (15 mg/Kg). Abbreviations: Ctx, Prefrontal Cortex; Str, Striatum; Hip, Hippocampus; N.D., not detected. | | | | |

In order to have a more precise estimation about BBB permeability to tadalafil, data from treated mice (see table 1) is reported as logBB in Table 2. BB is the ratio of brain versus serum concentration. Taking into account the values obtained in the three brain areas, the logBB for tadalafil is -0.89+/-0.09, which indicates that around 12% of tadalafil in bloodstream crosses the blood-brain barrier to reach the brain. In addition, considering values in the three brain regions, the compound distribution looks quite uniform.

**Table 2. Blood-brain barrier permeability, as logBB, for Tadalafil**

| Sample | logBB (Ctx) | logBB (Str) | logBB (Hip) | logBB (brain)^{a} |
|---|---|---|---|---|
| | | | | |
| n=6 | -0.89 *(0.16)^{b}* | -0.98 *(0.13)^{b}* | -0.81 *(0.17)^{b}* | -0.89 *(0.09)^{b}* |

| | | | | |
|---|---|---|---|---|
| a) logBB obtained as average value after considering logBB data from Ctx, Str and Hip; b) Standard Deviation value. | | | | |

### Effect of tadalafil and sildenafil on cognition in AD mice models

The Morris water maze (MWM) was used to assess the effect of sildenafil and tadalafil on the performance of the J20 mouse model of AD. The details of the administration of the compounds are given in Figure 1.

No significant differences were found among the groups during the training phase of the test (visible platform). Despite a 4-week treatment with sildenafil or tadalafil showed a trend to improve the escape latencies in the treated animals, no significant differences were detected in the hidden platform phase of the test (data not shown). It should be noted that the animals were quite old (20 month) and, therefore, the cognitive impairment was severe. For this reason, the treatment was prolonged to complete 10 weeks of treatment and the MWM was repeated. In this case, PDE5-inhibitor-treated animals were able to correctly learn the platform location, whereas untreated animals failed to do so. Moreover, in the last day of the test the scores of animals treated with tadalafil were even better than those of non-transgenic mice, which have slightly declined cognition capabilities as those expected for a geriatric/aged population. Furthermore, the cognitive improvement was higher in the group of tadalafil than in the group of sildenafil (statistically significant differences p<0.05) (Figure 2).

As a putative measurement of memory retention, mice swam in the pool with the platform removed at the beginning of days 7 and 9 of the invisible platform phase (Figure 3). The percentage of time spent in the right quadrant was analyzed as output of memory retention ability. Transgenic animals treated with saline showed lower scores than those attributed to randomness (25%) and significantly lower than those of non-transgenic animals. Both sildenafil- and tadalafil-treated animals displayed retention times higher than 25%, thus demonstrating a positive effect of the inhibitors in spatial memory.

Taken together the results using the MWM indicate that a 10-week treatment with sildenafil or tadalafil markedly improves the performance of 22-month old J20 animals, whose cognition is severely affected. In fact, the performance of saline-treated 22-month old J20 animals was very poor.

### Tadalafil effect on amyloid- and pTau-based pathology in AD mice models

Amyloid plaques in the brain of J20 mice (Mucke *et al.*, 2000) were still present in animals treated with PDE5 inhibitors. As shown in Figure 4, amyloid deposits in hippocampus were similar in saline or inhibitor-treated animals, suggesting that cognitive improvement does not depend on plaque disappearance.

To evaluate tau-based pathology hippocampal and cortical brain samples from non-transgenic or J20 animals treated with PDE5 inhibitors or saline were analyzed using the PHF-1 anti-p-Tau Ser-396/404 antibody. As shown in Figure 5 animals receiving tadalafil treatment did show a decrease in pTau levels, and such effect was much more marked than in animals treated with sildenafil. Importantly, some animals treated with tadalafil did not exhibit pTau pathology (Figure 5). These findings of lesser pTau pathology in animals treated with tadalafil than with sildenafil were confirmed using a different anti-pTau antibody that recognizes phosphorylation in Ser202 and Thr205 (Figure 6).

The decrease in Tau pathology in mice hippocampus treated with tadalafil or sildenafil was confirmed by Western blotting using the AT8 antibody (Figure 7). Tadalafil treatment was even more effective than sildenafil in reducing pTau levels (Figure 7).

Similar results were obtained using PHF1 antibody. Again, the effect was more marked in tadalafil-treated mice (Figure 8).

pTau levels depend on the activity of various kinases and phosphatases. Since GSK3β, among other kinases, phosphorylates tau at Ser-202 (AT8 immnunoreactivity) (Hanger *et al*., 1992; Mandelkow *et al.*, 1992) the levels of its Ser 9-phosphorylated inactive form (pGSK3β-Ser-9) were analyzed. Hippocampal levels of the inactive GSK3β form were lesser in transgenic (20-22 month-old) than in non-transgenic animals. Sildenafil and tadalafil treatment significantly increased the pGSK3β-Ser9 levels, being higher the effect of tadalafil (Figure 9).

Active Akt (pAkt) is able to phosphorylate GSK3β in the Ser-9 residue, which is crucial for kinase inactivation (Figure 10). Phosphorylation of GSK3β in Ser-9 prevents phosphorylation of tau in Ser-202.

The level of pAkt in the hippocampus was reduced in transgenic versus non-transgenic animals. This reduction was reverted by sildenafil and tadalafil treatment. Again the effect of tadalafil was higher than that of sildenafil (Figure 11).

Synaptic plasticity proteins play a fundamental role in cognitive function. Synaptophysin, GluR2/3 and PSD95 are examples of this type of proteins. The hippocampal expression levels of these proteins are markedly decreased in J20 mice compared to age-matched non-transgenic mice (Mucke *et al.,* 2000; Simón *et al.*, 2009). Western blot assays showed that sildenafil or tadalafil treatment recovered the low expression levels of synaptophysin (figure 12), GluR2/3 (Figure 13) and PSD95 (Figure 14) in the hippocampus of J20 mice to levels higher than that of non-transgenic wild type mice.

### Effect of tadalafil and sildenafil on cognition in aged wild type animals

The MWM and the FC test were used to assess the effect of 5-weeks treatment with sildenafil or tadalafil (15 mg/Kg) on the performance of aged (12-14-month-old) wild type animals (C57/b6xSJL). In both, the MWM (Figure 15) and the FC tests (24h and 7 days after training) (Figure 16), the treatment with tadalafil improved learning ability and memory retention of aged wild type animals. The effect of sildenafil was however only observed in the FC test (24h after training) (Figures 16).

### Discussion

PDE5 inhibition effect on cognition restoration was studied using a well-established AD mouse model. Two PDE5 inhibitors were used, sildenafil and tadalafil. Sildenafil has been already proved as an efficacious drug in improving memory in AD mouse models. In those studies tadalafil was used but no effect was demonstrated (Puzzo et al., 2009). They assumed that the lack of effect of tadalafil is due to its inability to cross the blood brain barrier. This was reflected in their subsequent patent applications. Here we show that tadalafil crosses the blood brain barrier and is equally and/or more effective than sildenafil in attenuating/restoring the cognitive memory impairments of AD mouse model (J20 mice). Furthermore, we show that tadalafil is able to restore synaptic plasticity parameters and pTau levels, as those found in non-transgenic animals. Even more, tadalafil treatment is able to enhance memory capacity of aged wild type animals. For all the above indicated reasons, tadalafil has a therapeutic potential for taupathies and age-related cognitive impairment: from mild cognitive impairment to any type of dementia, including full/overt dementia as in Alzheimer's disease.

### METHODS

### Determination of tadalafil in serum and brain samples

Tadalafil determination has been performed by liquid chromatography - mass spectrometry (HPLC-MS: Agilent 1100 with XCT Plus ion trap mass analyzer equipped with an electrospray ionization (ESI) source - G1948A model (see Tables 3 and 4).

**Table 3. Parameters of cromatography separation**

| Column | Zorbax Eclipse XDB-CN (4,6x150 mm, 5 µm) |
|---|---|
| Mobile phase | A: Ammonium formate pH=3. |
| | B: Acetonitrile |
| | Flow ramp: 0-2 min, 40:60 (A:B); gradient 10:90 (A:B) 1 |
| | min; keep 10:90 (A:B) for 4 min; gradient 40:60 (A:B) 1 |
| | min; keep 40:60 (A:B) for 2 min. |
| | Flux: 0.6 ml min⁻¹ |

**Table 4. Ion trap LC/MS acquisition parameters**

| Segment | Compound | Retention time (min) | Ion [M+H]⁺ | Transition MRM *(m*/*z)* | Fragmentation amplitude *(V)* |
|---|---|---|---|---|---|
| | Desmetilsildenafil | 4.9 | 461 | 461→283 | 0.61 |
| 0-6.1 min | Sildenafil | 5.3 | 475 | 475→283 | 0.65 |
| | Desetilsildenafil | 5.6 | 447 | 447→299 | 0.51 |
| 6.1-10 min | Tadalafil | 6.6 | 390 | 390→268 | 0.64 |

For analysis samples were prepared as follows:
- 100 µl of serum were treated with 300 µl of 1% formic acid in acetonitrile. Mixture was vortexed for 5 minutes. The supernatant after centrifugation was filtered using a HybridSPE^{™} (Supelco, Bellefonte, USA) cartridge and the eluate was dried under an air stream. The powder was reconstituted in 100 µl of 50:50 (v/v) ammonium formate (pH=3):acetonitrile. After centrifugation the supernatant was used for analysis.
- 100 mg of brain sample were treated with 600 µl of 1% formic acid in acetonitrile. The mixture was sonicated for 10 seconds and centrifuged for 5 minutes. The supernatant after centrifugation was filtered using a HybridSPE^{™} (Supelco, Bellefonte, USA) cartridge and the eluate was dried under an air stream. The powder was reconstituted in 100 µl of 50:50 (v/v) ammonium formate (pH=3):acetonitrile. After centrifugation the supernatant was used for analysis.

Calibration in the matrix itself led to the linearity ranges and detection limits indicated in table 5.

**Table 5**

| **COMPOUND** | **SERUM** | | | **BRAIN SAMPLES** | | |
|---|---|---|---|---|---|---|
| | Linearity range (µM) | Loq (µM) | Lod (µM) | Linearity range (µM) | Loq (µM) | Lod (µM) |
| **SILDENAFIL** | 0,016-3,253 | 0,016 | 0,005 | 0,016-0,407 | 0,016 | 0,005 |
| **DESMETILSILD.** | 0,006-3,879 | 0,006 | 0,002 | 0,006-0,483 | 0,006 | 0,002 |
| **DESETILSILD.** | 0,020-3,991 | 0,020 | 0,006 | 0,020-0,498 | 0,020 | 0,006 |
| **TADALAFIL** | 0,009-5,835 | 0,009 | 0,003 | 0,009-0,730 | 0,009 | 0,003 |

### Mouse model and treatment

Transgenic mice overexpressing human amyloid precursor protein (hAPP) with the Swedish (K670N/M671L) and Indiana (V717F) familial AD mutations under control of the PDGF β-chain promoter were used (J20 line) (Mucke *et al*, 2000). The mice were on an inbred C57BL/6J genetic background.

Based on effectiveness and safety, the dose of sildenafil citrate (Viagra®; from Pfizer, New York, NY, USA) used in patients with erectile dysfunction was 25 to 100 mg/day. The dosage of sildenafil we used in the transgenic AD mice model was 15 mg/kg/day, which extrapolated to a human male of 70 kg of weight is equivalent to 85 mg/day using the BSA-based dose calculation (Reagan-Shaw *et al.,* 2008). In order to compare the effects in cognition of both PDE5 inhibitors, the same dose was used for tadalafil: 15 mg/kg/day.

To test the long-term effect of sildenafil and tadalafil in cognitive function related to AD, 19-20 month old female J20 mice were treated once daily with sildenafil (15 mg/kg, orally), tadalafil or 0.9% NaCl "saline" solution for 10 weeks. Results were compared with those obtained from age-matched negative littermates (controls). To test the effect of the PDE5 inhibitors in cognition capacity related to aging, a second treatment was carried out using 10-12 month-old non-transgenic (wild type) mice on an inbred C57/B6xSJL genetic background. In this case the mice were treated once daily with sildenafil (15 mg/kg, orally), tadalafil (15 mg/kg, orally), or saline solution for 5 weeks.

All procedures were carried out in accordance with European and Spanish regulations (86/609/CEE; RD1201/2005). This study was approved by the Ethical Committee of the University of Navarra (no. 018/05). Behavioral studies were carried out during light time (from 9 am to 2 pm).

### Morris Water Maze Test (MWM)

The MWM test was used to evaluate spatial memory as previously described (Hsiao *et al.,* 1996; Reed *et al.,* 2010; Ricobaraza *et al.,* 2009). After treatments, groups of animals underwent spatial reference learning and memory testing in the MWM. The water maze was a circular pool (diameter 1.2 m) filled with water maintained at 20 °C and made opaque by the addition of non-toxic white paint. Mice were trained for three consecutive days (8 trials/day) swimming to a raised platform (visible-platform). No distal visible cues were present during this phase. The same platform location was used for all visible-platform sessions and was changed for the invisible-platform training (submerged 1 cm beneath the surface) conducted over 8 consecutive days (4 trials/day) with all visible distal cues present in this phase. In both visible- and hidden-platform versions, mice were placed pseudo-randomly in selected locations, facing toward the wall of the pool to eliminate the potentially confounding contribution of extramaze spatial cues. Each trial was terminated when the mouse reached the platform or after 60 seconds, whichever came first. To test the retention, three probe trials were performed at the beginning of 4th, 7th, and the last day of the test (day 9). In the probe trials the platform was removed from the pool, and the percentage of time spent in the quadrant where the platform was previously set was recorded. All trials were monitored by a camera above the center of the pool connected to a SMART-LD program (Panlab S.L., Barcelona, Spain) for subsequent analysis of escape latencies, swimming speed, path length and percent time spent in each quadrant of the pool during probe trials. All experimental procedures were performed blind to groups.

### Fear conditioning test

After running the MWM, all the animals were trained in the fear conditioning test. The conditioning procedure was carried out in a StartFear system (Panlab S.L.) that allows recording and analysis of the signal generated by the animal movement through a high sensitivity Weight Transducer system. The analogical signal is transmitted to the FREEZING and STARTLE software modules through the load cell unit for recording purposes and posterior analysis in terms of activity/immobility. The conditioning box is housed inside a soundproof chamber, which minimized external stimulation during the conditioning and retention tests. The box was provided with a house light that supplied dim illumination and with a floor grid through which foot shocks could be administered. On training day, the mice were placed in the conditioning chamber for 2 minutes before the onset of a tone at 2800 Hz, 85 dB (conditioned stimulus, CS), which lasted for 30 seconds. The last 2 seconds of the CS was paired with a 0.3 mA foot shock (unconditioned stimulus, US). Thirty seconds after the paired CS-US, mice were returned to their cage. Twenty-four hours later the mice were replaced in the conditioning chamber; after two minutes of exposure, the tone starts for a period of 2 minutes and freezing time was assessed during the 4 minutes (no differences were found in the freezing behavior with or without the tone). Freezing behavior was defined as the lack of movement except for breathing for at least 2 seconds, and was analyzed to give the percentage time freezing during exposure to the chamber. The conditioning apparatus was controlled by the experimenter with specific software (Packwin, Panlab S.L.) running on a PC computer.

Twenty four hours after the fear conditioning test, animals (n=5, per group) were sacrificed and the brains removed for biochemical studies. The cortex and hippocampus were dissected, homogenized, and processed as described below for subsequent western blot analysis. Under xylazine/ketamine anesthesia, animals (n=4 per group) were perfused transcardially with saline and 4% paraformaldehyde in phosphate buffer (PB). After perfusion, brains were removed, post-fixed in the same fixative solution for 1 h at room temperature and cryoprotected in 30% sucrose solution in PB overnight at 4 °C. Microtome sections (30-mm-thick) were cut coronally, collected free-floating and stored in 30% ethylene glycol, 30% glycerol, and 0.1M PB at - 20 °C until processed.

### Immunohistochemistry

Floating tissue sections comprising hippocampal formation were processed for 6E10 immunostaining following the protocol previously described by Ricobaraza *et al.* (2009). Briefly, brain sections were incubated in blocking solution (PBS containing 0.5% Triton X-100, 0.1% BSA and 2% normal goat serum) for 2 hours at room temperature. After washing, sections were incubated in 70% formic acid for 7 minutes to expose the epitope. Sections were incubated with the 6E10 antibody (against amino acids 1-17 of Aβ peptide, 1:200, Chemicon) for 24 hours at 4 °C, washed with PBS and incubated with the secondary antibody (Alexa Fluor 488 goat anti-mouse highly cross-absorbed, Molecular Probes, Eugene, Oregon, USA, 1:400) for 2 hours at room temperature, protected from light. Fluorescence signals were detected with confocal microscope LSM 510 Meta (Carl Zeiss, Germany); objective Plan-neofluar 40x/1.3 oil DIC. Sections were evaluated in Z-series (0.4 µm steps) using LSM 510 Meta software.

For p-tau staining, slides were incubated in blocking solution (3% milk in TBS) and incubated overnight with primary antibody AT8 (1:50 v/v, Pierce, Woburn, MA, USA) or PHF1 (1:100 v/v, Dr. Peter Davies, Albert Einstein College, Bronx, NY, USA) at 4 °C. Sections were washed with PBS and incubated with the secondary antibody (Alexa Fluor 488 goat anti-mouse highly cross-absorbed, Molecular Probes, Eugene, Oregon, USA, 1:400) for 2 hours at room temperature, protected from light. Fluorescence signals were detected with confocal microscope LSM 510 Meta (Carl Zeiss, Germany); objective Plan-neofluar 40x/1.3 oil DIC. Sections were evaluated in Z-series (0.4 microm steps) using LSM 510 Meta software.

### Production of protein extracts

Total tissue homogenates were obtained by homogenizing the hippocampus in a cold lysis buffer with protease inhibitors (0.2 M NaCl, 0.1 M HEPES, 10% glycerol, 200 mM NaF, 2 mM Na₄P₂O₇, 5 mM EDTA, 1 mM EGTA, 2 mM DTT, 0.5 mM PMSF, 1 mM Na₃VO₄, 1 mM benzamidine, 10 µg/ml leupeptin, 400 U/ml aprotinin), centrifuged at 14,000 x g 4 °C for 20 minutes and the supernatant was aliquoted and stored at -80 °C. Total protein concentrations were determined using the BioRad Bradford protein assay (BioRad Laboratories).

### Immunoblotting

Protein samples were mixed with an equal volume of 2 x Laemmli sample buffer, resolved onto SDS-polyacrylamide gels and transferred to nitrocellulose membrane. The membranes were blocked with 5% milk, 0.05% Tween-20 in PBS or TBS followed by overnight incubation with the following primary antibodies: mouse monoclonal anti-p-tau AT8 (1:1000, Pierce Biotechnology, Inc. Rockford, USA), mouse monoclonal anti-p-tau PHF1 (1: 1000 v/v, Dr. Peter Davies, Albert Einstein College, Bronx, NY, USA), mouse monoclonal anti tau (1:5000, clone Tau46, Sigma-Aldrich, St. Luis, MO, USA), rabbit polyclonal anti-pGSK3-Ser9 (1:1000, Cell Signalling Technology, Beverly, MA), rabbit polyclonal anti-pAkt-Ser473 (1:1000, Cell Signalling Technology), rabbit monoclonal anti-Akt (1:1000, Cell Signalling Technology), rabbit polyclonal anti-GSK3 (1:1000, Santa Cruz Biotechnology, Santa Cruz, CA), rabbit polyclonal anti-GluR2/3 (1:1000, Chemicon, Temecula, CA, USA), mouse monoclonal anti-PSD95 (1:1000, Chemicon); mouse monoclonal anti-synaptophysin (1:1000, Chemicon); mouse monoclonal anti-actin (1:2000, Sigma-Aldrich, St. Louis, MO, USA) and mouse monoclonal anti-tubulin (1:10000, Sigma-Aldrich) in the corresponding buffer. Following two washes in PBS/Tween-20 or TBS/Tween-20 and one PBS or TBS alone, immunolabeled protein bands were detected by using HRP-conjugated anti-rabbit or anti-mouse antibody (Santa Cruz; dilution 1:5000) following an enhanced chemiluminescence system (ECL, GE Healthcare Bioscience, Buckinghamshire, UK), and autoradiographic exposure to Hyperfilm^{™}ECL (GE Healthcare Bioscience). Quantity OneTM software v.4.6.3 (Bio-Rad) was used for quantification.

### Data analysis and statistical procedures

The results were processed for statistical analysis using SPSS package for Windows, version 15.0 (SPSS, Chicago, IL, USA). Unless otherwise indicated, results are presented as mean ± SEM. In the MWM and fear conditioning test, escape latencies during training were analyzed using one-way analysis of variance (ANOVA) followed by Scheffé post hoc test. In the MWM, Friedman's test was performed to determine the intra group comparisons over trials. Biochemical data were analyzed using Kruskal-Wallis test followed by Mann Whitney post hoc test. Student's t test was used in case two groups were compared.

### References

Almeida CG, et al. Beta-amyloid accumulation in APP mutant neurons reduces PSD-95 and GluR1 in synapses. Neurobiol.Dis. 2005 Nov;20(2):187-198.
Baek SB, et al. The PDE5 inhibitor, tadalafil, improves depressive symptoms, ameliorates memory impairment, as well as suppresses apoptosis and enhances cell proliferation in the hippocampus of maternal-separated rat pups. Neurosci.Lett. 2011;488:26-30.
Blanpied TA and Ehlers MD. Microanatomy of dendritic spines: emerging principles of synaptic pathology in psychiatric and neurological disease. Biol.Psychiatry 2004 Jun 15;55(12):1121-1127.
Brunet A, et al. Transcription-dependent and -independent control of neuronal survival by the PI3K-Akt signaling pathway. Curr.Opin.Neurobiol. 2001 Jun; 11(3):297-305.
Forgue ST, et al. Tadalafil pharmacokinetics in healthy subjects. Br.J.Clin.Pharmacol. 2006 Mar;61(3):280-288.
Hardy J. A hundred years of Alzheimer's disease research. Neuron 2006 Oct 5;52(1):3-13. Hong M, et al.. Lithium reduces tau phosphorylation by inhibition of glycogen synthase kinase-3. J.Biol.Chem. 1997 Oct 3;272(40):25326-25332.
Knobloch M, and Mansuy IM. Dendritic spine loss and synaptic alterations in Alzheimer's disease. Mol.Neurobiol. 2008 Feb;37(1):73-82.
Ko IG, et al. Tadalafil improves short-term memory by suppressing ischemia-induced apoptosis of hippocampal neuronal cells in gerbils. Pharmacol.Biochem.Behav. 2009; 91:629-635.
Kulkarni SK, and Patil CS. Phosphodiesterase 5 enzyme and its inhibitors: update on pharmacological and therapeutical aspects. Methods.Find.Exp.Clin.Pharmacol. 2004 Dec;26(10):789-799.
Lovestone S, et al. Alzheimer's disease-like phosphorylation of the microtubule-associated protein tau by glycogen synthase kinase-3 in transfected mammalian cells. Curr.Biol. 1994 Dec 1;4(12):1077-1086.
Morris R. Developments of a water-maze procedure for studying spatial learning in the rat. J.Neurosci.Methods 1984 May;11(1):47-60.
Mucke L, et al. High-level neuronal expression of abeta 1-42 in wild-type human amyloid protein precursor transgenic mice: synaptotoxicity without plaque formation. J.Neurosci. 2000 Jun 1;20(11):4050-4058.
Muñoz-Montano JR, et al. Lithium inhibits Alzheimer's disease-like tau protein phosphorylation in neurons. FEBS Lett. 1997 Jul 14;411(2-3):183-188.
Puerta E, et al. On the mechanisms underlying 3,4-methylenedioxymethamphetamine toxicity: the dilemma of the chicken and the egg. Neuropsychobiology 2009;60(3-4):119-129.
Puerta E, et al. Sildenafil protects against 3-nitropropionic acid neurotoxicity through the modulation of calpain, CREB, and BDNF. Neurobiol.Dis. 2010 May;38(2):237-245. Puzzo D, et al. Phosphodiesterase 5 inhibition improves synaptic function, memory, and amyloid-beta load in an Alzheimer's disease mouse model. J.Neurosci. 2009 Jun 24;29(25):8075-8086.
Ricobaraza A, et al. Phenylbutyrate ameliorates cognitive deficit and reduces tau pathology in an Alzheimer's disease mouse model. Neuropsychopharmacology 2009 Jun;34(7):1721-1732.
Scheff SW, et al.. Synaptic alterations in CA1 in mild Alzheimer disease and mild cognitive impairment. Neurology 2007 May 1;68(18):1501-1508.
Selkoe DJ. Cell biology of protein misfolding: the examples of Alzheimer's and Parkinson's diseases. Nat.Cell Biol. 2004 Nov;6(11):1054-1061.
Small GW, et al. Diagnosis and treatment of Alzheimer disease and related disorders. Consensus statement of the American Association for Geriatric Psychiatry, the Alzheimer's Association, and the American Geriatrics Society. JAMA 1997 Oct 22-29;278(16):1363-1371.
Terry K. Impaired physicians. Speak no evil? Med.Econ. 2002 Oct 11;79(19):110-2, 114, 117.
Zhang L, et al. Tadalafil, a long acting type 5 PDE isoenzyme inhibitor, improves neurological functional recovery in a rat model of embolic stroke. Brain Research 2006; 1118:192-198.

## Claims

1. Tadalafil, pharmaceutically acceptable salts and solvates thereof, for use in the prevention or treatment of dementia in a geriatric patient.

2. Tadalafil for use according to claim 1, wherein said tadalafil is administered at a dose between 20 and 100 mg once a day.

3. Tadalafil for use according to claim 1, wherein said tadalafil is administered at a dose between 20 and 40 mg once a day.

4. Tadalafil for use according to any one of claims 1-3, wherein said tadalafil is administered orally.

5. Tadalafil for use according to any one of claims 1-4, wherein dementia is presenile or senile.

6. Tadalafil for use according to any one of claims 1-5, wherein dementia courses with tauopathy.

7. Tadalafil for use according to any one of claims 1-6, wherein dementia is of the Alzheimer's type.

8. Tadalafil for use according to any one of claims 1-6, wherein dementia is a moderate dementia of the Alzheimer's type.

9. Tadalafil for use according to any one of claims 1-6, wherein dementia is a severe dementia of the Alzheimer's type.

10. Tadalafil, pharmaceutically acceptable salts and solvates thereof, for use in the prevention or treatment of Alzheimer's disease in a geriatric patient.

11. Tadalafil for use according to claim 10, wherein said tadalafil is administered at a dose between 20 and 100 mg once a day.

12. Tadalafil for use according to claim 10, wherein said tadalafil is administered at a dose between 20 and 40 mg once a day.

13. Tadalafil for use according to any one of claims 10-12, wherein said tadalafil is administered orally.

14. Tadalafil for use according to any one of claims 10-13, wherein Alzheimer's disease is of moderate grade.

15. Tadalafil for use according to any one of claims 10-13, wherein Alzheimer's disease is of moderate to severe grade.

16. Tadalafil for use according to any one of claims 10-13, wherein Alzheimer's disease is of severe grade.
